# EUROPEAN PATENT SPECIFICATION

(11) **EP 1 670 823 B1**
(45) Date of publication and mention of the grant of the patent: **02.10.2013**
(21) Application number: 04768739.7
(22) Date of filing: 04.10.2004
(51) Int. Cl.: C07K 14/705, A61K 38/17, C07K 14/74, C07K 19/00, C12N 5/12, C12N 15/12, C12N 15/62, C12Q 1/68, G01N 33/68, A61K 47/48

(54) **CHIMERIC MHC PROTEIN AND OLIGOMER THEREOF FOR SPECIFIC TARGETING**
CHIMÄRES MHC-PROTEIN UND OLIGOMER DAVON FÜR SPEZIFISCHES TARGETING
PROTEINE MHC CHIMERE ET SON OLIGOMERE POUR CIBLAGE SPECIFIQUE

(30) Priority: 06.10.2003 GB 0323324
(43) Date of publication of application: 21.06.2006
(73) Proprietor: ProImmune Limited, Littlemore Park Oxford OX4 4SS (GB)
(72) Inventor: SCHWABE, Nikolai, Franz, Gregor, Oxford OX2 6BP (GB)
(74) Representative: Cremer, Christian
(86) International application number: PCT/GB2004/004199
(87) International publication number: WO 2005/035567

(56) References cited:
- WO-A-01/90198
- WO-A-98/18943
- WO-A-99/42597
- WO-A-02/102299
- WO-A-2004/018520
- WO-A2-02/055992
- US-A1- 2002 082 411
- US-A1- 2003 044 415
- MÜLLER K M ET AL: "Protein fusions to coiled-coil domains", METHODS IN ENZYMOLOGY, vol. 328, 2000, - 2000, pages 261-282, XP008025980,
- HOLLER N ET AL: "Development of improved soluble inhibitors of FasL and CD40L based on oligomerized receptors", JOURNAL OF IMMUNOLOGICAL METHODS , ELSEVIER SCIENCE PUBLISHERS B.V., AMSTERDAM, NL, vol. 237, no. 1-2, 2000, - 2000, pages 159-173,

## Description

The present invention relates to a chimeric MHC protein, an expression cassette encoding the same, a vector, an oligomer of said chimeric protein, and various uses of the oligomeric MHC complex.

### Background of the Invention

Major Histocompatibility Complex (MHC) molecules, which are found on the cell surface in tissues, play an important role in presenting cellular antigens in the form of short linear peptides to T cells by interacting with T cell receptors (TCRs) present on the surface of T cells.

It has been established that isolated or recombinant forms of MHC-peptide molecules are useful for detecting, separating and manipulating T cells according to the specific peptide antigens these T cells recognize. It has also been understood that the interaction between MHC molecules and TCRs across cell surfaces is multimeric in nature and that the affinity of a single MHC molecule for a given TCR is generally low. As a consequence, there has been an effort to develop multimeric forms of isolated or recombinant MHC-peptide molecules to make such molecules more useful in the applications described above.

European Patent Application EP 812 331 discloses a multimeric binding complex for labeling, detecting and separating mammalian T cells according to their antigen receptor specificity, the complex having the formula (α-β-P)ₙ, wherein (α-β-P) is an MHC peptide molecule, n is ≥ 2, α comprises an a chain of a MHC I or MHC II class molecule, β comprises a β chain of an MHC protein and P is a substantially homogeneous peptide antigen. The MHC peptide molecule is multimerised by biotinylating the C terminus of one of the a or β chain of the MHC molecule and coupling of MHC monomers to tetravalent streptavidin/avidin or by providing a chimeric protein of an MHC molecule which is modified at the C terminus of one of the α or β chain to comprise an epitope which is recognised by a corresponding antibody that serves as a multimerising entity. The document further teaches use of the MHC oligomers for detecting, labeling and separating specific T cells according to their TCR specificity.

European Patent Application EP 665 289 discloses specific peptides, MHC molecules binding these peptides, and oligomers obtained by crosslinking of the respective MHC molecules having the specific peptide bound to them. Oligomerisation is achieved by using chemical crosslinking agents or by providing MHC chimeric proteins comprising an epitope, which is recognised by an immunoglobulin such as IgG or IgM. The MHC molecules may comprise a label and may be used for labeling, detecting, and separating T cells according to their specific receptor binding, and may eventually be employed in therapy of humans.

WO 93/10220 discloses a chimeric MHC molecule, comprising the soluble part of an MHC molecule, which can be either class I or class II MHC fused to an immunoglobulin constant region. The MHC portion of the molecule comprises complementary α and/or β chains and a peptide is bound in the respective binding grooves of the MHC molecules. Due to the presence of the dimeric immunoglobulin scaffold these chimeric MHC-Ig molecules undergo self-assembly into a dimeric structure.

In other research the oligomerisation domain of cartilage oligomeric matrix protein (COMP) has been used as a tool for multimerising several proteins in the past. COMP has been described and characterised by Efimov and colleagues (see e.g. Proteins: Structure, Function, and Genetics 24:259-262 (1996)). COMP is a pentameric glycoprotein of the thrombospondin family. Self-assembly of the protein to form pentamers is achieved through the formation of a five-stranded helical bundle that involves 64 N-terminal amino acid residues of the protein. The amino acid sequence of the oligomerisation domain has been disclosed by Efimov et al., FEBS Letters 341:54-58 (1994).

WO 00/44908 discloses chimeric proteins that contain anti-angiogenic portions of TSP-1, TSP-2, endostatin, angiostatin, platelet factor 4 or prolactin linked to a portion of the N-terminal region of human cartilage oligomeric matrix protein (COMP) thus allowing for the formation of pentamers. The document is predominantly concerned with exploiting the anti-angiogenic effect mediated by the resulting chimeric proteins. According to this disclosure the chimeric protein should promote correct folding of the TSP-domains contained therein, so that they better mimic the natural proteins than peptides that are based on the TSR sequence.

WO 02/055992 discloses self-multimerising MHC fusion proteins and oligomeric complexes thereof.

WO99/64464 describes targeting of MHC molecules to a specific cell type by linking the molecules to a target specific antibody molecule. Where the target cell is a diseased, foreign or malignant cell, this method may be used to promote the lysis of the target cell by T cells in the immune system. In this context it is possible to make use of pre-existing T cell responses by employing MHC-peptide complexes that are specific for endemic persistent infections found in many healthy individuals such as Epstein Barr Virus infections and Cytomegalovirus infections. Alternatively, the target cell may be used as an antigen presenting cell, to promote the proliferation of specific T cell clones that may kill other diseased, foreign or malignant cells or afford protective immunity against such cells to the patient.

As an example MHC-anti-CD20 antibody complexes are disclosed as suitable for targeting MHC complexes to CD20 positive Daudi cells, which may be useful in killing CD20 positive Daudi Burkitt's lymphoma cells.

Following on from this work Savage et al. in British Journal of Cancer (2002) 86, 1336-1342 have shown that it is possible to elicit antigen-specific syngeneic T cell responses against viral and tumor derived antigens using an two-step targeting system wherein biotinylated recombinant HLA-class I peptide complexes loaded with relevant antigenic peptides were attached to the surface of B cells via an anti-CD20 single chain variable domain antibody-streptavidin fusion protein.

Concerns remain, however, regarding the potentially unacceptable toxicity and immunogenicity of using non-human content in the MHC-targeting complexes of the prior art, such as the streptavidin content in the assembly of the MHC-streptavidin-scFv complex, especially where therapeutic use *in vivo* is envisaged.

In addition producing MHC multimers that rely on the biotin-streptavidin interaction involves a substantial number of process steps, including several rounds of protein purification, and a biotinylation reaction that can lead to significant loss of active material. Further, controlling the biotinylation efficiency of monomeric MHC subunits and quality of the final multimeric product is difficult. Finally, the tetrahedral arrangement of the biotin/streptavidin-complex puts certain sterical constraints and limitations on the obtained MHC multimer.

The non-prepublished international application WO 2004/018520 filed August 14,2003 and assigned to the present applicant discloses an oligomeric MHC complex comprising at least two chimeric proteins, said chimeric proteins comprising a first section derived from an MHC peptide chain or a functional part thereof and a second section comprising an oligomerising domain derived from an oligomer-forming coiled-coil protein, wherein formation of the oligomeric MHC complex occurs by oligomerisation at the oligomerisation at the oligomerising domain of the chimeric proteins, and wherein at least two of the first sections are derived from the same MHC peptide chain. The disclosure of this document is incorporated herein by way of reference in its entirety. The disclosed complex lacks, however, the possibility of specific targeting to a desired cell type.

It is the object of the present invention to overcome the above drawbacks and other disadvantages of the prior art and to provide a multimeric MHC peptide complex that is easy to manufacture with a uniformly high valency of MHC peptide components that are available simultaneously for binding to T cell receptors and a protein sequence allows for minimizing non-human content and that can be targeted to the surface of target cells with high affinity and specificity.

### Summary of the Invention

The above disadvantages and drawbacks of the prior art are overcome by the oligomeric MHC complex according to claim 1. At least two chimeric proteins comprising a first section derived from an MHC peptide chain or a functional part thereof and a second section comprising an oligomerisation domain derived from an oligomer-forming coiled-coil protein are used to form an oligomeric MHC complex, which complex further allows for its targeting to a certain cell or tissue.

A first aspect of the present invention therefore concerns an oligomeric MHC complex comprising at least two chimeric proteins, said chimeric proteins comprising a first section derived from an MHC peptide chain or a functional part thereof and a second section comprising an oligomerising domain derived from an oligomer-forming coiled-coil protein, said complex further comprising attaching means for selectively attaching said MHC complex to a target cell, wherein formation of the oligomeric MHC complex occurs by oligomerisation at the oligomerisation at the oligomerising domain of the chimeric proteins, and wherein at least two of the first sections are derived from the same MHC peptide chain. The oligomerising domain is from the pentamerisation domain of the cartilage oligomeric matrix protein.

Preferably the first section of the chimeric proteins is derived from the extra-cellular part of the MHC class I or II a chain or the extra-cellular part of the MHC class I or II β chain.

Preferably the oligomerising domain comprised in the second section in at least one of the chimeric proteins is derived from the pentamerisation domain of the human cartilage oligomeric matrix protein (COMP).

At least one of the chimeric proteins, preferably all of them, may further comprise a first linker between the MHC peptide chain and the oligomerising domain.

The attaching means may be linked to an oligomerisation domain of one or more of the chimeric proteins forming the complex, preferably one of them. The link may generally be made by any appropriate means, but occurs preferably via a peptide bond or a polypeptide linker. Accordingly, in a preferred embodiment the attaching means is comprised in a third section of at least one of the chimeric proteins of the complex.

Said attaching means comprises a linking polypeptide with high specific affinity for a target cell specific molecule on the surface of the target cell. Most preferably at least one linking polypeptide is bound to at least one of the chimeric proteins of the complex by a peptide bond or a polypeptide linker and thereby forms a third section of said chimeric protein.

Preferably said linking polypeptide comprises at least a portion of an antibody, preferably a monoclonal antibody, raised against said target specific molecule. More preferably said linking polypeptide comprises a variable domain and optionally constant domain(s) of the binding portion derived from a monoclonal antibody. The oligomeric MHC complex may additionally comprise the complementary variable (and constant) domain(s) of the monclonal antibody.

In another embodiment said linking polypeptide is a single chain variable fragment (scFv) derived of a monoclonal antibody.

Preferably the first section of the chimeric protein(s) of the invention complex is located N-terminal of the second section and the third section, if present, is located C-terminal of said second section.

At least one of the chimeric proteins of the complex may further comprise one or more domains selected from the group consisting of a further linker, a tagging domain and a purification domain. These can be present on the same or different chimeric proteins forming the complex of the invention. The oligomeric MHC complex may also comprise a label.

The oligomeric MHC complex as described above may further comprise the complementary MHC peptide chains to at least two of the chimeric proteins to form functional MHC binding complexes. Preferably it further comprises peptide bound to the MHC portions of the complex in the groove formed by the MHC α1 and α2 domains for class I complexes or the MHC α1 and β1 domains for class II complexes. More preferably the peptide is substantially homogenous.

Most preferably the peptide is selected from the group consisting of (a) a peptide against which there is a pre-existing T cell immune response in a patient, (b) a viral peptide e.g. occurring in the group of Epstein Barr Virus, Cytomegalovirus, Influenza Virus, (c) an immunogenic peptide of immunogens used in common vaccination procedures, and (d) a tumour specific peptide, a bacterial peptide, a parasitic peptide or any peptide which is exclusively or characteristically presented on the surface of diseased, infected or foreign cell.

In one embodiment of the oligomeric MHC complex the allotype of the MHC molecules in the complex is different from the allotype of a patient.

In one embodiment of the oligomeric MHC complex said target cell is selected from the group of an antigen presenting cell, a cell carrying a surface marker in high copy number, and a CD20 positive cell.

In a second aspect the invention relates to the use of the complex to amplify antigen-specific or allospecific T cells *in vivo* or *in vitro*.

In a third aspect the invention relates to a chimeric protein comprising
- a first section derived from an MHC peptide chain or a functional part thereof; and
- a second section comprising an oligomerising domain derived from an oligomer-forming coiled-coil protein which coiled-coil protein oligomerises by alignment of at least two substantially identical versions of the polypeptide chain from which the oligomerising domain is derived; and
- a third section comprising a variable domain derived from an antibody which antibody is specific for a target molecule present on the surface of a target cell. Further preferred embodiments are analogous to those set out above.

Preferably the chimeric protein comprises an oligomerising domain derived from the pentamerisation domain of the human cartilage oligomeric matrix protein (COMP). More preferably the oligomerising domain comprises and preferably consists of the amino acid 1 to 128, preferably 20 to 83, most preferably 20 to 72 of COMP.

In a fourth aspect the invention relates to a recombinant expression cassette comprising a promoter sequence operably linked to a nucleotide sequence coding for a chimeric protein as defined above.

In a fifth aspect the invention relates to a vector comprising the above recombinant expression cassette.

In a sixth aspect the invention relates to a pharmaceutical or diagnostic composition, comprising an oligomeric MHC complex as defined above, optionally in combination with a pharmaceutically acceptable carrier.

The invention further relates to the use of the above complex for preparing a pharmaceutical composition for immunizing a patient against a disease or condition which is characterized by the presence in the patient's body of cells displaying disease associated MHC binding peptides on the surface thereof that are associated with the said disease or condition and wherein the oligomeric MHC complex comprises binding peptides which are similar to or substantially the same as the said disease associated MHC binding peptides.

The invention finally relates to the use of the above complex for preparation of a pharmaceutical composition for causing an immune-response in a patient to destroy unwanted target cells by directing an immune response against said target cells.

### Description of the Drawings

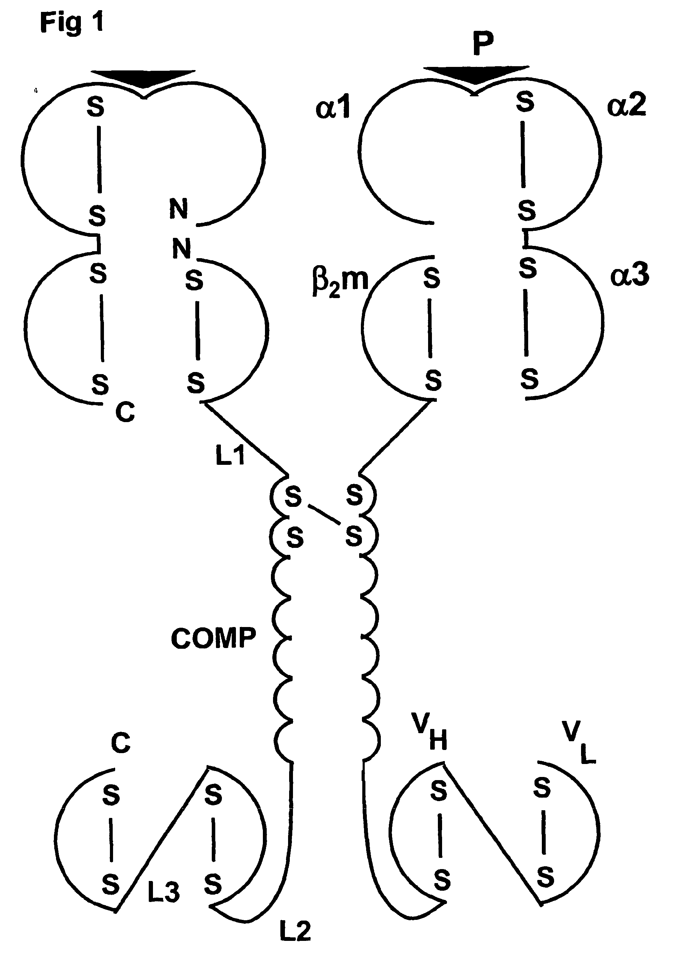

Fig. 1 is a schematic drawing of an oligomeric MHC class I complex of the invention. The figure shows two out of all subunits of the fully assembled oligomeric class I chimeric complex. As shown β2-microgobulin (β2m) is fused to the coiled oligomerisation domain (OD) of an oligomer-forming coiled-coil protein and spaced from this domain by a first linker (L1). A further linker (L2) is provided at the C terminal end of the domain, followed by the variable region of the heavy chain V_{H} of an immunoglobulin molecule which is specific for a desired cell-surface molecule. V_{H} is linked at its C-terminal end to the N-terminus of the variable region of the immunoglobulin light chain V_{L} via a further third linker (L3). The complementary MHC class I α chain having the domains α1, α2, and α3, is assembled with β2m and antigenic peptide (P). Disulphide bonds (S-S), stabilizing the MHC portion of the complex are indicated. Further disulphide bonds within the oligomerisation domain are also shown, illustrating the specific case where the oligomerising domain is derived from COMP, which will assemble into a stable pentamer, wherein these disulphide bonds link each pair of the COMP domains in the pentamer. Similarly the immunoglobulin variable region heavy and light chains are stabilized by disulphide bonds. The amino and carboxyl termini of the respective α and β chains in the complex are indicated by N and C, respectively.

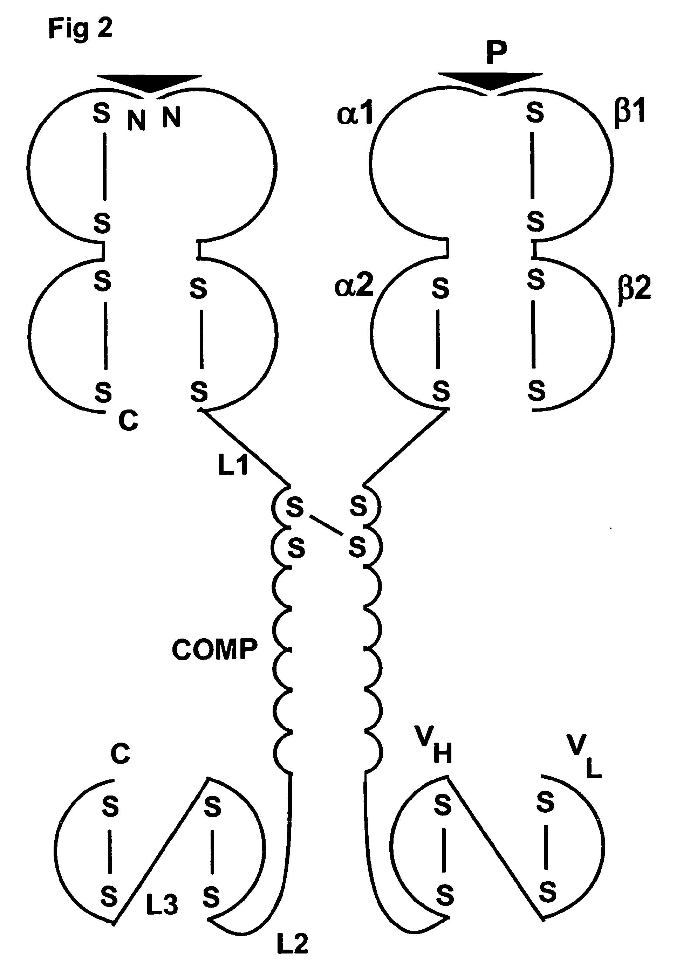

Fig. 2 is a schematic drawing of an oligomeric MHC class II complex of the invention. The figure generally follows the nomenclature of Fig. 1 with the difference that (β2m) is replaced by the MHC class II α chain, having the domains α1, α2, fused to the oligomerising domain via the linker (L1). Here the α chain is assembled with its complementary MHC class II β chain, having the domains β1, β2.

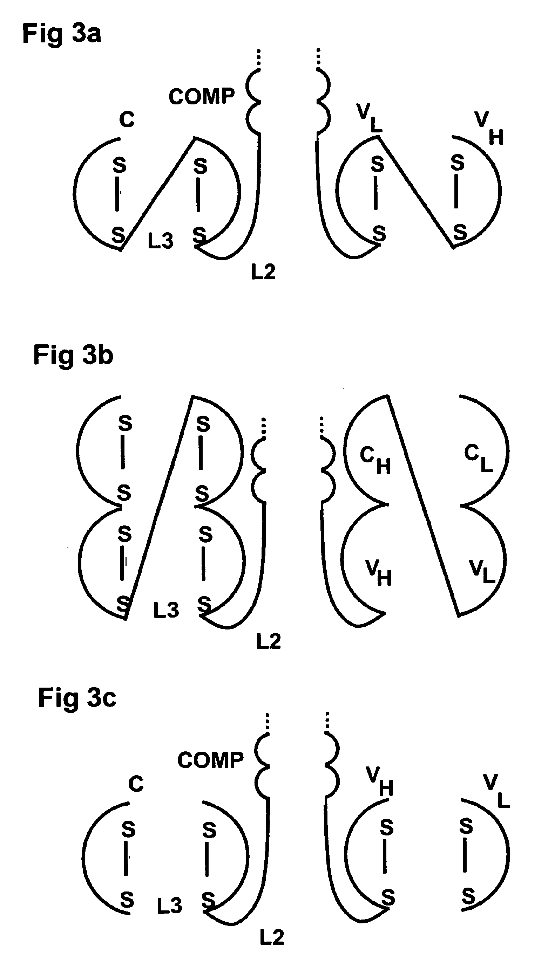

Fig. 3 shows alternative embodiments of the immunoglobulin portion of the multimeric complex. In Fig. 3a the heavy and light chains of the immunoglobulin molecule are interchanged. In Figure 3b, both the variable and constant domains of the binding portion of the immunoglobulin molecule are included in the construct and the linking can occur between the constant region of the first chain and the variable region of the second chain. In Fig. 3c the linker between the immunoglobulin heavy and light chains is omitted and the final molecule is formed by non-covalent assembly of the immunoglobulin chains, either during protein expression or by means of a separate refolding reaction.

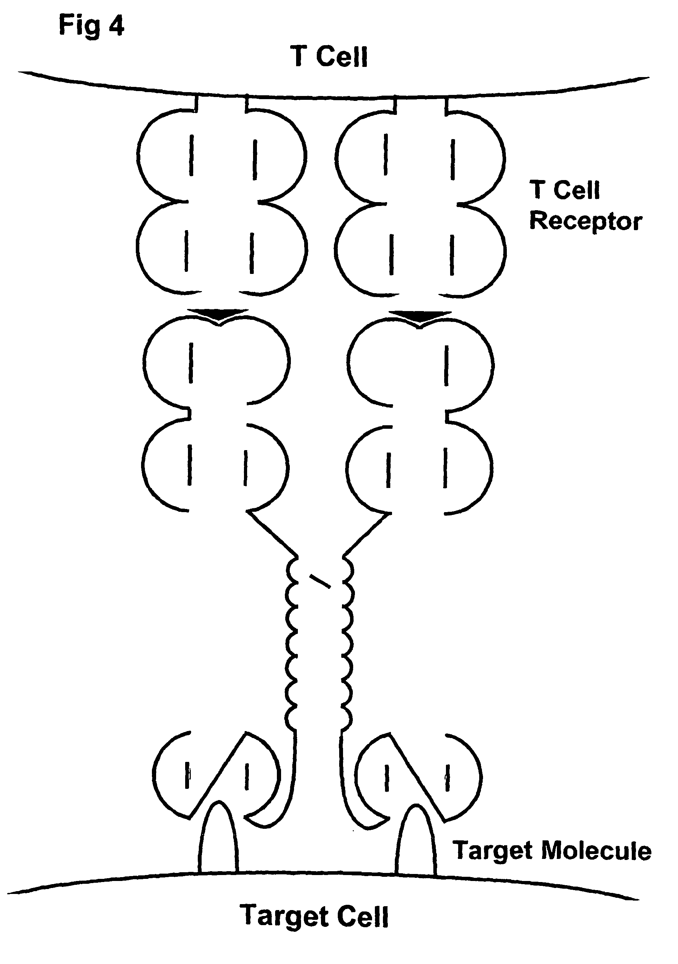

Fig. 4 shows simultaneous multivalent binding of the oligomeric MHC-complex of the invention to a target cell via a target molecule present on the surface of the target cell, for which the immunoglobulin portion of the oligomeric-MHC complex is specific, and to the T cell receptors on the surface of an antigen specific T cell which engage the binding portion of the MHC-peptide complex molecules. The domain structure of the T cell receptors is also indicated, including disulphide bonds stabilizing these molecules.

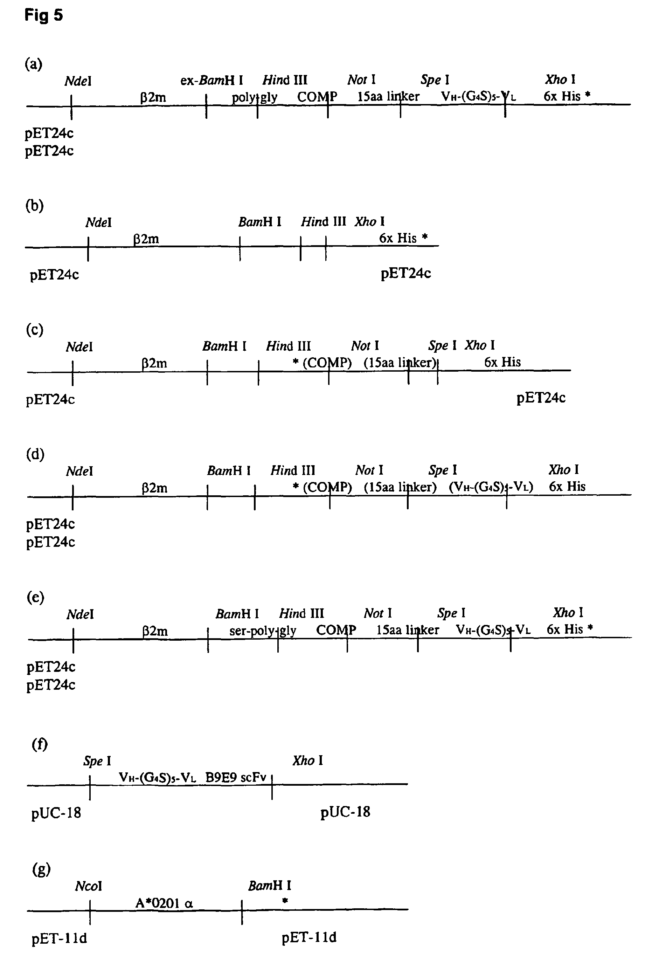

Fig. 5 shows a set of schematic maps, including restriction sites, illustrating (a) pETBMS05: the β2m-COMP-scFv expression construct, as well as (b)-(e) pETBMS01-04: β2m-COMP-scFv intermediate molecular cloning constructs, and (f) pUCB9E9: the B9E9 scFv synthetic gene construct. Finally (g) illustrates pETA2sol: the HLA-A*0201 α chain expression construct, as described in detail later on. Stop codons are indicated by the '*' symbol for each construct in (a)-(g).

### Detailed Description of the Invention

The oligomeric MHC complex of the invention allows for overcoming the disadvantages and drawbacks of the prior art. More specifically, the inventors have found that the above oligomeric MHC complex can have a substantially higher affinity to a T cell receptor than an oligomer obtained by e.g. tetramerising the MHC complexes by coupling through biotin and scFv-streptavidin as in Savage et al. in British Journal of Cancer (2002) 86, 1336-1342.

Without wishing to be bound by theory it is believed that this increase in affinity is achieved when three or more MHC molecules are arranged substantially in the same plane with all binding faces oriented in the same direction. In contrast, because the tetrameric streptavidin-coupled complex has a tetrahedral arrangement, at best only three MHC binding domains are available simultaneously for contacting the T cell surface. In the same manner the immunoglobulin portions of the oligomeric complex are all disposed at the same end of the complex allowing for highly multivalent binding to target molecules that are present on the surface of target cells.

The meaning of an "oligomer-forming coiled-coil protein" within the scope of the present invention is a protein comprising an oligomerising domain. Said domains comprise two or more polypeptide subunits that may or may not be identical. The subunit of two or more of such oligomerisation domains assemble with one another such that each subunit in the oligomerising domain assumes a substantially helical conformation in the assembled state, wherein the subunits in the oligomerisation domain are arranged \ along an identifiable axis, wherein the oligomerising domain has two identifiable opposite ends along said axis, and wherein at least two of the polypeptide subunits have the same amino-to-carboxyl orientation along said axis. Corresponding oligomerisation domains and oligomer-forming coiled-coil proteins are known in the art as e.g., cited in the introductory portion hereof.

The oligomerising domain may be derived from a suitable oligomer-forming coiled-coil protein of any species. Preferably, the MHC molecule is fused to an oligomerisation domain of a human version oligomer-forming coiled-coil protein, in which case unwanted immune responses and/or rejection reactions are minimised in situations where the complex is to be administered to humans. For in-vitro-applications non-human domains may, however, as well as used.

Examples for oligomer-forming coiled-coil proteins include various types of collagen, triple coiled-coil domains of C-type lectins, such as mannose binding protein (MBP); C1q, myosin, leucine zippers such those occurring in p53, GCN4, bacteriophage P22 Mnt repressor; and the trombospondin family proteins such as COMP. Preferably the oligomerisation domain is derived from the cartilage oligomeric matrix protein COMP. More preferably, the MHC molecule is fused to an oligomerisation domain of the human version of COMP for the reasons described above.

The number of chimeric proteins (n) comprised on the oligomeric MHC complex of the invention will typically depend on the type of oligomerisation domain the second section of the chimeric proteins is derived from and can in general be 2 or more, preferably n = 2 to 10, most preferably n = 3 or 5. If the second section of the chimeric proteins to be oligomerised is e.g. derived from the pentamerisaion domain of the COMP this number will be five such that the oligomer will be a pentamer (n = 5). In case these oligomerisation domains are derived from collagen, this number will be three (n = 3).

The first aspect of the invention relates to an oligomeric MHC complex comprising chimeric proteins comprising a first section derived from an MHC peptide chain or a functional part thereof, a second section comprising an oligomerisation domain of an oligomer-forming coiled-coil protein, and an attaching means for attaching and targeting the complex to a target cell. Although the MHC peptide chain of the first section preferably forms the N terminal part of the chimeric protein, whereas the oligomerising domain of the second section is located in the C terminal region thereof, both sections can in general be in any order. The preferred arrangement will, however, allow the MHC part to maintain its functional characteristics for all cases. The attaching means needs not form part of any of the oligomerised chimeric proteins, but preferably it does as set out below.

The term "chimeric protein" as used herein means a single peptide protein, the amino acid sequence of which is derived at least in part from two different naturally occurring proteins or protein chain sections, in this case an MHC peptide and a peptide substantially comprising at least a significant proportion of an oligomerising domain. With the term "a functional part thereof" as used herein, a part of a peptide chain is meant, which still exhibits the desired functional characteristics of the full-length peptide it is derived from. For MHC therefore a peptide chain is meant, which, if necessary, when completed or assembled with the complementary MHC peptide chain thereof, is capable of binding an antigenic peptide. With the term "complementary" MHC peptide chain the respective other peptide chain of a naturally occurring MHC complex is meant. The complementary chain to an α chain of the MHC complex is the β chain and *vice versa.*

According to a first embodiment the MHC peptide chain in the chimeric protein is the extra-cellular part of the MHC class I or II α chain. According to another embodiment the MHC peptide chain is the extra-cellular part of an MHC class I or II β chain. The MHC proteins may be from any vertebrate species, e.g. primate species, particularly humans; rodents, including mice, rats, hamsters, and rabbits; equines, bovines, canines, felines; etc. Of particular interest are the human HLA proteins, and the murine H-2 proteins. Included in the HLA proteins are the class II subunits HLA-DPα, HLA-DPβ, HLA-DQα, HLA-DQβ, HLA-DRα and HLA-DRβ, and the class I proteins HLA-A, HLA-B, HLA-C, and β2 -microglobulin. Included in the murine H-2 subunits are the class I H-2K, H-2D, H-2L, and the class II I-Aα, I-Aβ, I-Eα and 1-Eβ, and β2-microglobulin. Amino acid sequences of some representative MHC proteins are referenced in EP 812 331.

In a preferred embodiment, the MHC peptide chains correspond to the soluble form of the normally membrane-bound protein. For class I subunits, the soluble form is derived from the native form by deletion of the transmembrane and cytoplasmic domains. For class I proteins, the soluble form will include the α1, α2 and α3 domains of the α chain. For class II proteins the soluble form will include the α1 and α2 or β1 and β2 domains of the α chain or β chain, respectively.

Not more than about 10, usually not more than about 5, preferably none of the amino acids of the transmembrane domain will be included. The deletion may extend as much as about 10 amino acids into the α3 domain. Preferably none of the amino acids of the α3 domain will be deleted. The deletion will be such that it does not interfere with the ability of the α3 domain to fold into a functional disulfide bonded structure. The class I β chain, β2m, lacks a transmembrane domain in its native form, and does not have to be truncated. Generally, no class II subunits will be used in conjunction with class I subunits.

The above deletion is likewise applicable to class II subunits. It may extend as much as about 10 amino acids into the α2 or β2 domain, preferably none of the amino acids of the α2 or β2 domain will be deleted. The deletion will be such that it does not interfere with the ability of the α2 or β2 domain to fold into a functional disulfide bonded structure.

One may wish to introduce a small number of amino acids at the polypeptide termini, usually not more than 25, more usually not more than 20. The deletion or insertion of amino acids will usually be as a result of the requirements in cloning, e.g. as a consequence of providing for convenient restriction sites or the like, and to manage potential steric problems in the assembly of the molecules. In addition, one may wish to substitute one or more amino acids with a different amino acid for similar reasons, usually not substituting more than about five amino acids in any one domain.

In an alternative embodiment the MHC class I or class II α and β chains may be joined as a single chain construct via an appropriate linker, such as a glycine-serine linker. The construct may be α-linker-β in the direction of N-terminus to C-terminus or β-linker-α in the direction of N-terminus to C-terminus, whichever is more convenient for the application in question.

According to the present invention the MHC peptide chain comprised in the first section of a chimeric protein as defined above is fused, preferably at its C terminal end, to the oligomerisation domain of an oligomer-forming coiled-coil protein as defined above. Where the pentamerising domain of COMP is used as the oligomerising domain, this domain comprises, more preferably consists of the N terminal amino acids 1 to 128, preferably 20 to 83, most preferably 20 to 72 of said protein as discussed in the prior art section of this invention disclosure. With regard to numbering of amino acids, reference is made to Efimov et al., FEBS Letters 341:54-58 (1994). Further, similar to the MHC part of the chimeric protein of the invention, this domain can be altered by amino acid substitution, deletion or insertion, as long as the self-assembly of the oligomerising domain is not impaired.

Fusion of both peptide chains, MHC and the oligomerising domain, respectively, may be direct or, as is shown in Figures 1 and 2, may include a first linker (L1) connecting and spacing apart the MHC peptide and the oligomerising domain (OD) in the chimeric protein. Joining by use of a linker is preferred. In general such (polypeptide) linker will comprise not more than 30, preferably not more than 26 amino acids. Suitable linkers are known in the art and include e.g. immunoglobulin hinge regions, or glycine-serine repeat sequences.

The oligomeric MHC complex of the invention formed by oligomerising the appropriate number of chimeric proteins as defined above further comprises an attaching means for selectively attaching (and targeting) said MHC complex to a target cell. The attaching means can in general be affixed to the complex at any suitable site and by any suitable means, provided it does not interfere with the oligomerisation, and the MHC-TCR binding, and does not separate under conditions of its use. Typically the attaching means will thus be affixed to the complex by covalent bonding either directly or through a (polypeptidic or other) linker, e.g. conventional hydrocarbon spacers, or through specific binding pairs, such as antibody-epitope reactions or antibody-hapten reactions.

Preferably the attaching means is affixed to an oligomerisation domain of one or more, and preferably to each one of the chimeric proteins. Again this affixation may be by any appropriate means but preferably occurs through a peptide bond or a polypeptide linker. Most preferably in such an embodiment the attaching means will thus be itself a linking peptide. It may in this case be comprised in and preferably form a third section of the chimeric protein of the invention.

This third section may be attached to either the N- or C-terminal end of the oligomerisation domain, and is preferably attached to the opposite end with regard to attachment of the MHC-section. The chimeric protein comprising the third section may lack a first section.

In a preferred embodiment, as is shown in Figs. 1-3, the chimeric protein of the invention is further fused, preferably at the C terminal end and optionally via a second polypeptide linker (L2) such as described above for α1 and exemplified by a Glycine Serine linker (GGGGS)₅, to a linking polypeptide with a high specific affinity for a cell surface molecule on a target cell. Examples for suitable linking peptides are portions the polypeptide chain of an antibody binding domain such as the immunoglobulin variable region light chain V_{L} or variable heavy chain V_{H}, which may be associated with their natural immunoglobulin domain counterparts, or an antibody fragment such as a single chain variable fragment (scFv) which is capable of binding specifically to target molecules on the surface of target cells, such as cell surface receptors. Presence of the linking polypeptide allows the chimeric proteins and complexes of the invention to be targeted to cell surfaces in vivo and in vitro for research, diagnostic and therapeutic purposes.

One or more of the chimeric proteins of the complex may optionally further include, preferably at its C terminal end, one or more of a fourth linker (L4), a biotinylation peptide (BP) and a tagging domain and/or a purification domain (TD) in either order. In a preferred embodiment the chimeric protein of the invention includes all three of the above domains in this order. In another embodiment these domains may be present on differing chimeric proteins in the oligomeric MHC complex. In general the fourth linker will comprise not more than 25, preferably not more than 20 amino acids and can be the same as detailed for the first linker above. The above additional domains are preferably present on those, not comprising the attaching means.

Where a purification domain is to be optionally included in one or more of the chimeric proteins of the complex the skilled worker will know of several possible sequences, including, e.g. a hexahistidine sequence.

The tagging domain optionally included in one or more of the chimeric proteins of the complex can be any domain which allows for labeling of the protein. Preferably the tagging domain or the chimeric protein includes a label. This label can be included in the domain itself such as an epitope recognised by an antibody or a light detectable or radioactive label. Preferably, the label is selected from the group consisting of fluorescent markers, such as such as FITC, phycobiliproteins, such as R- or B-phycoerythrin, allophycocyanin, Cy3, Cy5, Cy7, a luminescent marker, a radioactive label such as ¹²⁵I or ³²P, an enzyme such as horseradish peroxidase, or alkaline phosphatase e.g. alkaline shrimp phosphatase, an epitope, a lectin or biotin/streptavidin.

Where the label is itself a protein, the polypeptide chain of the protein used for labeling can be fused to the chimeric protein in question, preferably at its C terminus, to the label protein. For example a fluorescent protein such as a green fluorescent protein (GFP), or a subunit of a phycobiliprotein could be used. GFP chimeric protein technology is well known in the art. Chimeric proteins comprising a suitable domain from a phycobiliprotein is described, for example in WO 01/46395.

Alternatively the label may be attached at a specific attachment site provided in the tagging domain. For example, the tagging domain may include a recognition site for the biotin protein ligase BirA to allow for site-specific biotinylation of the tagging domain and hence recognition by streptavidinlavidin. Suitable recognition sequences for BirA are well known in the art. Similarly a lectin may be attached, or any other site-specific enzymatic modification may be made by means of incorporating an amino acid recognition sequence for a modifying enzyme into the amino acid sequence of the chimeric proteins of the invention. Other possible types of enzymatic modification are described in EP812 331. Alternatively labeling can be achieved by binding of a suitably labeled antibody, or antibody fragment, such as a labeled F(ab) fragment to an epitope on the tagging domain or elsewhere on the molecule.

According to the first aspect thereof the present invention relates to an oligomeric MHC complex formed by oligomerising through self-assembly of the oligomerising domain of an oligomer-forming coiled-coil protein in the second section of the above chimeric proteins. Oligomerisation of the oligomerising domain typically occurs spontaneously and results in stable oligomers of the chimeric proteins. Typically, the oligomeric complex will consist of several identical monomeric subunits (at least in their MHC part, depending on the oligomerisation domain and presence of any of the above additional domain(s)). If desired, however, two or more chimeric proteins differing in their MHC portion may be admixed before oligomerisation. Thereby heterogeneous oligomers may be obtained. Generally, however, at least two of the chimeric proteins in each complex will comprise a first section derived from the same MHC α or β subunit. For n > 2 the oligomer may contain at least two of the chimeric proteins in each complex which comprise a first section derived from the same MHC molecule. Preferably all chimeric proteins of the complex comprise a first section derived from the same MHC molecule.

Preferably the oligomerising domain of COMP is used and results in spontaneous assembly of stable pentamers of the chimeric protein.

The oligomeric MHC complex may further comprise the complementary MHC peptide chain(s) to form functional MHC binding complexes as discussed above and may also comprise a peptide bound in the groove formed by the MHC α1 and α2 domains for class I MHC complexes or the MHC α1 and β1 domains for class II MHC complexes. Preferably the peptide is substantially homogeneous.

The antigenic peptides will be from about 6 to 14 amino acids in length for complexes with class I MHC proteins, and usually about 8 to 11 amino acids. The peptides will be from about 6 to 35 amino acids in length for complexes with class II MHC proteins, usually from about 10 to 20 amino acids. The peptides may have a sequence derived from a wide variety of proteins. In many cases it will be desirable to use peptides, which act as T cell epitopes. The epitope sequences from a number of antigens are known in the art. Alternatively, the epitope sequence may be empirically determined by isolating and sequencing peptides bound to native MHC proteins, by synthesis of a series of putative antigenic peptides from the target sequence, then assaying for T cell reactivity to the different peptides, or by producing a series of MHC-peptide complexes with these peptides and quantification the T cell binding. Preparation of peptides, including synthetic peptide synthesis, identifying sequences, and identifying relevant minimal antigenic sequences is known in the art. In any case, the peptide comprised in the oligomeric MHC complex is preferably substantially homogeneous, meaning that preferably at least 80% of the peptides are identical, more preferably at least 90 % and most preferably at least 95%.

Preferably the peptide is selected from the group consisting of (a) a peptide against which there is a pre-existing T cell immune response in a patient, (b) a viral peptide e.g. occurring in the group of Epstein Barr Virus, Cytomegalovirus, Influenza Virus, (c) an immunogenic peptide of immunogens used in common vaccination procedures, and (d) a tumour specific peptide, a bacterial peptide, a parasitic peptide or any peptide which is exclusively or characteristically presented on the surface of diseased, infected or foreign cell.

In a second aspect the present invention relates to a chimeric protein itself, which can be oligomerised into the oligomeric MHC complex of the invention. The chimeric protein comprises a first section derived from an MHC peptide chain or a functional part thereof and a second section comprising an oligomerising domain derived from an oligomer-forming coiled-coil protein which coiled-coil protein oligomerises by alignment of at least two substantially identical versions of the polypeptide chain from which the oligomerising domain is derived. In other words, the oligomer-forming coiled-coil protein used to derive the oligomerising domain in the chimeric protein of the present invention may be a homo-oligomer or a hetero-oligomer. Typically, however, it will comprise two or more copies of substantially identical polypeptide chain sections in its oligomerising domain that are aligned when the oligomer is formed. Preferably oligomerising domain derived from the pentamerisation domain of COMP. The chimeric protein also comprises the above discussed third section comprising the linking polypeptide. Preferred embodiments are as discussed above with regard to the oligomeric MHC complex itself.

In a third aspect thereof the present invention relates to a recombinant expression cassette comprising a promoter sequence operably linked to a first nucleotide sequence coding for an MHC peptide chain or a functional part thereof, a second nucleotide sequence encoding the oligomerising domain of COMP and a third nucleotide sequence encoding the variable region of an immunoglobulin molecule. In a first embodiment the first nucleotide sequence encodes the MHC peptide chain of the extra-cellular part of the MHC class I or II α chain. In a second embodiment the first nucleic acid sequence encodes the MHC peptide chain of the extra-cellular part of the MHC class I or II β chain. The second nucleotide sequence encodes amino acids 1 to 128, preferably 20 to 83, most preferably 20 to 72 of COMP. Preferably the COMP sequence is derived from human COMP; and the third nucleotide sequence encodes the linking peptide. These are joined in frame in any desired order and may be spaced apart by sequences coding for the linkers, if any. Preferably the third sequence encodes the variable region heavy chain and the variable region light chain of the immunglobulin having the desired specificity. Coding sequences for the above optional domains, which are known themselves, may be added.

Generally, the nomenclature used herein and the laboratory procedures in recombinant DNA technology described are those well known and commonly employed in the art. Standard techniques are used for DNA and RNA isolation, amplification, and cloning. Generally enzymatic reactions involving DNA ligase, DNA polymerase, restriction endonucleases and the like are performed according to the manufacturer's specifications, using enzyme buffers supplied by the manufacturer. These techniques and various other techniques are generally performed as known in the art. Suitable nucleotide sequences for the MHC and the oligomer-forming coiled coil domains such as the COMP domain are known in the art as discussed hereinbefore.

The DNA constructs will typically include an expression control DNA sequence, including naturally-associated or heterologous promoter regions, operably linked to protein coding sequences. The expression cassette may further include appropriate start and stop codons, leader sequences encoding sequences and so on, depending on the chosen host. The expression cassette can be incorporated into a vector suitable to transform the chosen host and/or to maintain stable expression in said host.

The term "operably linked" as used herein refers to linkage of a promoter upstream from one or more DNA sequences such that the promoter mediates transcription of the DNA sequences. Preferably, the expression control sequences will be those eukaryotic or non-eukaryotic promoter systems in vectors capable of transforming or transfecting desired eukaryotic or non-eukaryotic host cells. Once the vector has been incorporated into the appropriate host, the host is maintained under conditions suitable for high-level expression of the nucleotide sequences, and the collection and purification of the chimeric proteins.

MHC-DNA sequences, DNA sequences for suitable oligomerising domains from an oligomer-forming coiled-coil protein, including that of COMP, and DNA sequences for the desired immunoglobulin domains can be isolated in accordance with well-known procedures from a variety of human or other cells. Traditionally, desired sequences are amplified from a suitable cDNA library, which is prepared from messenger RNA that is isolated from appropriate cell lines. Suitable source cells for the DNA sequences and host cells for expression and secretion can be obtained from a number of sources, such as the American Type Culture Collection (ATCC) or other commercial suppliers.

The nucleotide sequences or expression cassettes used to transfect the host cells can be modified according to standard techniques to yield chimeric molecules with a variety of desired properties. The molecules of the present invention can be readily designed and manufactured utilizing various recombinant DNA techniques well known to those skilled in the art. For example, the chains can vary from the naturally occurring sequence at the primary structure level by amino acid insertions, substitutions, deletions, and the like. These modifications can be used in a number of combinations to produce the final modified protein chain. In general, modifications of the genes encoding the chimeric molecule may be readily accomplished by a variety of well-known techniques, such as site-directed mutagenesis.

The amino acid sequence variants as discussed above can be prepared with various objectives in mind, including increasing the affinity of the molecule for target T cells, increasing or decreasing the affinity of the molecule for the respective CD4 or CD8 co-receptors interacting with class I or class II MHC complexes, for facilitating purification and preparation of the chimeric molecule or for increasing the stability of the complex, *in vivo* and *ex vivo.* The variants will, however, typically exhibit the same or similar biological activity as naturally occurring MHC molecules and retain and retain their oligomerising property of the corresponding naturally occurring oligomerising domain of the relevant oligomer-forming coiled-coil protein, respectively.

In addition, preparation of the oligomer is a straightforward process. For therapeutic uses this may be carried out in mammalian cell culture, e.g. in CHO, COS, or human cell lines. Alternatively other expression systems can be used for expressing the molecules of the present invention, such as insect cell culture, including baculovirus and *Drosophila melanogaster* expression systems, yeast expression systems, or prokaryotic expression systems such as *Escherichia coli (E. coli).* If expression is performed in a prokaryotic expression system, either soluble expression, i.e. directed into the cytoplasm or periplasm, or insoluble expression, i.e. into inclusion bodies is possible.

A typical vector for *E*. *coli* would be one of the pET family of vectors which combine efficient expression from bacteriophage T7 RNA polymerase within an inducible *lac* operon-based system. The vectors containing the nucleotide segments of interest can be transferred into the host cell by well-known methods, depending on the type of cellular host. For example, transformation into chemical- or electro-competent cells is commonly utilised for prokaryotic cells, whereas calcium phosphate treatment, cationic liposomes, or electroporation may be used for other cellular hosts. Other methods used to transfect mammalian cells include the use of Polybrene, protoplast chimeric, microprojectiles and microinjection.

The chimeric protein and the complementary MHC α or β subunits, respectively, may expressed as a single fusion construct as or be co-expressed and assembled as oligomeric complexes in the same cell. Alternatively these two components may be produced separately and allowed to associate *in vitro* in the presence of the antigenic peptide of choice to form stable oligomeric MHC peptide complexes. In the latter case mixing and association of the two components may occur either before or after oligomerisation of the chimeric peptide

The advantage of association of separate components *in vitro* is that oligomeric MHC-peptide complexes can be obtained with very high peptide homogeneity, e.g. greater than 95 % or even greater than 99%. Where the complexes are expressed as fully assembled molecules, the peptide of interest can be introduced into the complexes, either by culturing the expressing cells with medium containing the antigenic peptide of interest, or by exchanging the peptide of interest with peptides that have endogenously bound during expression *in vitro,* which is typically done by incubating the purified complex with excess peptide at low or high pH so as to open the antigen binding pocket (see e.g. WO 93/10220). The antigenic peptide can also be covalently bound using standard procedures such as photoaffinity labeling (see e.g. WO 93/10220).

Alternatively, the peptide may be directly linked or fused to the expression construct of the chimeric protein or its complementary α or β chain. A suitable linker between the peptide portion and the N terminus of the chimeric protein or its complementary α or β chain, such as a polyglycine repeat sequence may be provided. Including the peptide as a chimeric peptide in the expression construct will allow for expression and folding of the complete MHC-peptide oligomeric complex without further addition of antigenic peptide or peptide exchange.

Conditions that permit folding and association of the subunits and chimeric proteins *in vitro* are known in the art. Assembly of class I MHC peptide complexes as well as assembly of functional class II complexes is e.g. described in EP 812 331. As one example of permissive conditions, roughly equimolar amounts of solubilised α and β subunits are mixed in a solution of urea in the presence of an excess of antigenic peptide of interest. In the case of class II MHC molecules refolding is initiated by dilution or dialysis into a buffered solution without urea. Peptides are loaded into empty class II heterodimers at about pH 5 to 5.5 over about 1 to 3 days, followed by neutralization, concentration and buffer exchange. Oligomerisation of the complex should occur simultaneously with formation of the α - β - peptide complex and the folding and assembly of the immunoglobulin domains. Alternatively a pentameric α or β complex may be achieved in two stages: first allowing oligomerisation of the oligomerising domain and folding and assembly of the immunoglobulin domains, followed by further refolding with the complementary α or β MHC subunit in the presence of peptide according to the methods described above.

The assembled complex of the invention, or, initially the separate chimeric protein of the present invention and the complementary α or β subunit, can be purified according to standard procedures of the art, including ammonium sulfate precipitation, gel electrophoresis, column chromatography, including gel filtration chromatography, ion exchange chromatography, hydrophobic interaction chromatography, affinity chromatography, and the like.

The resulting peptide chain or oligomeric MHC complex of the present invention may be used therapeutically or in developing and performing assay procedures, immuno-stainings, and the like. Therapeutic uses of the oligomeric MHC complexes of the present invention require identification of the MHC haplotypes and antigens useful in treating a particular disease. In addition it may be necessary to determine the tissue types of patients before therapeutic use of the complexes according to the present invention, which is, however, a standard procedure well known in the art. The present invention is particularly suitable for treatment of cancers and infectious diseases, autoimmune diseases, and prevention of transplant rejection. Based on knowledge of the pathogenesis of such disease and the results of studies in relevant animal models, one skilled in the art can identify and isolate the MHC haplotype and antigens associated with a variety of such diseases.

In a fifth aspect the present invention therefore relates to a pharmaceutical or diagnostic composition, comprising the above oligomeric MHC complexes as defined above. Pharmaceutical compositions comprising the proteins are useful for, e.g. parenteral administration, i.e. subcutaneously, intramuscularly or intravenously.

In this aspect of the invention the oligomeric MHC complexes described here can be targeted to the surfaces of target cells *in vivo* and *in vitro.* In this manner the complexes can be used to stimulate either allospecific T cell responses against the target cells in question or generate peptide-antigen-specific T cell responses against the same MHC-peptide target on MHC-peptide targets presented on other cells.

The cell surface target molecules to which the oligomeric MHC complexes can bind may be tumour associated antigens, such as carcinoembryonic antigen, placental alkaline phosphatase, polymorphic epithelial mucin, human chorionic gonadotrophin, CD20, prostate specific antigen and others. Target specific monoclonal antibody expressing cell lines can be generated by methods well known in the art. Such cell lines can in turn be used as a cDNA source for cloning of the binding portions of these antibodies for fusion with the chimeric protein of the invention.

The target cell may be cultured *in vitro* where it can be cultured with cytotoxic T cells in order to activate and proliferate antigen-specific or allospecific T cells. Alternatively the target cells may be occurring in the patient *in vivo* and the complexes are administered to the patient. When targeted to cell types that do not possess co-stimulatory molecules the complexes may also be used in vitro or in vivo to ablate specific T cell responses, such as allospecific T cell responses in a transplantation setting or antigen-specific T cell responses in an autoimmune disease context by causing anergy of the specific T cell types.

Where the target cell is cultured *in vitro* it may be co-cultured with T cells that have been already pre-selected according to their antigen specificity, e.g. by means of fluorescence activated cell sorting with fluorochrome labeled MHC-peptide tetrameric complexes. Hence the complexes of the present invention can be used for example to purify expand allospecific or antigen-specific T cells and re-introducing these T cells to a patient autologously after expansion and/or other manipulation in cell culture in various disease situations.

The target cell may be a tumour cell or any diseased of foreign cell, which should be eliminated in a patient, such as a leukemia cell, or other cancer cell, a cell infected with a virus, such as HIV, hepatitis B or C virus, human papilloma virus, or a microbe or parasite.

Preferably the MHC-peptide combination to be used will be capable of producing a strong immune response in the patient. Accordingly the MHC molecule to be used may be mismatched to the tissue type of the patient which will produce strong allospecific immune responses, or it will be a tissue matched MHC molecule containing a viral or microbial peptide to which the patient is likely to already have a good immune response. Such peptides may be preferably derived from endemic persistent infections Influenza virus, Epstein Barr Virus, Cytomegalovirus or peptide from an organism against which the patient has likely had a prior vaccination.

In another embodiment the target cell is used as an antigen-presenting vehicle. It could be a natural antigen-presenting cell (APC). Alternatively, it could be a substitute cell, which carries a surface marker in high copy number, such as a healthy CD20 positive B cell, which expresses a high number of copies of CD20 on its surface. In this case the MHC complex can be targeted to the high-copy number surface marker to cause highly multivalent presentation of MHC complexes on the cell surface, which generates a powerful antigen-presenting vehicle. In either case the MHC complex will preferably comprise an antigenic peptide that is a viral peptide, bacterial peptide, parasitic, peptide or cancer specific peptide which is exclusively or typically presented by MHC molecules on the surface of diseased, malignant or foreign cells which are desirable to be eliminated in the patient. As a consequence, forming an antigen presenting vehicle with the oligomeric MHC complex of the invention as described above may be used *in vivo* or *in vitro* to generate an immune response against undesired cells which are different from the target cells.

The proliferation and differentiation of T cells stimulated with the complexes according to the invention may be improved and directed by simultaneously administering cytokines to the patient or adding such cytokines to the cell culture. Such cytokines may include interleukins, granulocyte monocyte colony stimulating factor (GM-CSF) and the like.

Compared to these prior art technologies of targeting MHC complexes to the cell surface of target cells the oligomeric MHC-peptide-antibody domain or fragment complexes of the present invention open the possibility of generating complexes with fully humanized content, very compact size, multimeric structure of both the antibody binding portion of the molecule and the MHC-peptide presentation portion of the molecule which are each positioned advantageously on opposite ends of the coiled-coil multimeric domain, which simultaneously afford high target binding affinity and high T cell binding affinity.

The compositions for parenteral administration will commonly comprise a solution of the chimeric protein or pentamer thereof dissolved in an acceptable carrier, preferably an aqueous carrier. A variety of aqueous carriers can be used, e.g. buffered water, 0.4 % saline, 0.3 % glycine and the like. These solutions are sterile and generally free of particulate matter. These compositions may be sterilised by conventional, well-known sterilization techniques. The compositions may contain pharmaceutically acceptable auxiliary substances as required to approximate physiological conditions such as pH adjusting and buffering agents, toxicity adjusting agents and the like, for example sodium acetate, sodium chloride, potassium chloride, calcium chloride, sodium lactate, etc. The concentration of the chimeric protein in these formulations can vary widely, i.e. from less than about 1 pg/ml, usually at least about 0.1 mg/ml to as much as 10 - 100 mg/ml and will be selected primarily based on fluid volumes, viscosities, etc. in accordance with the particular mode of administration selected.

A typical pharmaceutical composition for intramuscular injection could be made up to contain 1 ml sterile buffered water, and 0.1 mg of oligomer complex protein. A typical composition for intravenous infusion could be made up to contain 250 ml of sterile Ringer's solution, and 10 mg of oligomer complex protein. Actual methods for preparing parenterally administrable compositions will be known or apparent to those skilled in the art.

The chimeric proteins or oligomeric MHC complexes of this invention can be lyophilised for storage and reconstituted in a suitable carrier prior to use. This technique has been shown to be effective and commonly used lyophilization and reconstitution techniques can be employed. It will be appreciated by those skilled in the art that lyophilization and reconstitution can lead to varying degrees of activity loss and that use levels may have to be adjusted to compensate.

Due to their enhanced affinity for specific T cell receptors, the oligomeric MHC complexes according to the present invention can allow for improved drug potency, better serum half-life and also improved pharmacokinetics, the latter presumably being due to increased molecular mass.

Due to the possibility of expressing the chimeric proteins and complementary MHC α or β portions comprised in the oligomeric MHC complexes of the invention in non-eukaryotic systems they are further easy to make at high yields, and can be expressed as sub-components, which can subsequently be refolded with one another in the presence of a homogeneous population of antigenic peptide.

These and other advantages are available to the skilled worker from the foregoing description. The following examples are given for the purpose of illustration only and shall not be construed as limiting the present invention in any way.

### Examples

The following is a detailed example for cloning, expressing, and purifying a pentameric class I MHC complex, which comprises a chimeric fusion between β2m, COMP, and an anti-CD20 scFv. The chimeric β2m-COMP-scFv protein is expressed in insoluble inclusion bodies in *E*. *coli* and subsequently assembled as pentameric β2m-COMP-scFv *in vitro.* The pentameric class I MHC peptide complex is then formed in a second refolding reaction by combining β2m-COMP-scFv pentamers and the human MHC class I α molecule known as HLA-A*0201, in the presence of an appropriate synthetic binding peptide representing the T cell antigen. In this example, a well characterized antigen derived from Epstein-Barr virus BMLF1 protein, GLCTLVAML (a.a. 289-297), is used.

### 1. Molecular cloning of the β2m-COMP-scFv construct

The strategy involves the sequential cloning into pET-24c vector of β2m, yielding a construct referred to as pETBMS01, followed by the sequential insertion of the oligomerisation domain of cartilage oligomeric matrix protein (COMP) (pETBMS02), and the sequence encoding the anti-CD20 scFv, yielding a construct referred to as pETBMS03. Next, a polyglycine linker is cloned in between β2m and COMP, resulting in the construct pETBMS04, and finally, a serine-residue is removed by site-directed mutagenesis, which serine residue precedes the poly-glycine linker, to give the final β2m-COMP-ScFv/pET-24c construct, referred to as pETBMS05 (see also Fig. 5 (a)). Removal of the serine residue is carried out to avoid steric hindrance when the β2m molecule associates with the MHC class I αchain protein.

Incidentally the cloning procedure outlined below will result in retention of the pET-24c vector encoded histidine tag at the C-terminus of the expressed protein. This may offer additional options for detection and purification of the protein construct. Alternatively an untagged version may be obtained by carrying out site-directed mutagenesis to insert a stop codon in front of the tag region.

### 1.1. Cloning β2m into the pET-24c vector

### Source of β2m

The extracellular portion of β2m comprises of 99 amino acids (equivalent to Ile1-Met99 of the mature protein) encoded by 74 bp-370 bp of the DNA sequence. This region of the β2m sequence is amplified from a normal human lymphocyte cDNA library, by polymerase chain reaction (PCR) using the primers BM_F (1 µM) and BM_R (1 µM) which incorporate *Nde*I and *BamH*I restriction sites, respectively. (Sigma-Genosys, see Appendix I for primer sequences). Amplification is carried out using a proof-reading DNA polymerase (*Pfx*, Invitrogen) (1.25 U) in *Pfx* amplification buffer (as supplied by the manufacturer), supplemented with 2 mM MgSO₄ and 0.2 mM dNTPs following standard thermal cycling conditions. Step 1: 94°C, 4 min; Step 2: 94°C, 1 min; Step 3: 55°C, 1 min; Step 4: 72°C, 30 sec; Step 5: cycle to Step 2, 34x, Step 6: 72°C 10 min.

### Restriction enzyme digestion of β2m and pET-24c

β2m PCR product is purified from the above reaction mix using a QIAquick PCR purification kit according to the manufacturer's instructions (Qiagen). 200 ng of purified PCR product and 1 µg pET-24c vector (Novagen) are each digested with *Bam*H I (10 U) and *Nde* I (10 U) restriction enzymes (New England Biolabs, NEB) for 4 h at 37°C, in accordance with the manufacturer's instructions. The digested fragments are separated by horizontal gel electrophoresis in 1 % agarose (Bioline), and purified using a QIAEX II gel extraction kit, according to the manufacturer's instructions (Qiagen).

### Ligation of β2m into pET-24c (pETBMC01)

The gel-purified insert and vector DNA are ligated at a 1:3 molar ratio (vector:insert, 50 ng: 7.5 ng) using T4 DNA ligase (5 U; Bioline), in T4 DNA ligase buffer (as supplied) for 16 hrs at 16°C.

### Transformation of pETBMC01 into E. coli host strain XL1-Blue

The ligation mixtures and appropriate controls are subsequently transformed into XL1-Blue strain competent *E. coli* cells, according to the manufacturer's instructions (Stratagene). Successful transformants are selected by plating the cells on Luria-Bertani (LB) agar plates containing 30 µg/ml kanamycin, and incubating overnight at 37°C.

### PCR screening of transformants

A selection of single colonies from the bacterial transformation plates are screened by PCR with T7 promoter (1 µM) and T7 terminator (1 µM) primers (Sigma Genosys, see Appendix I for primer sequences), which are complementary to regions of the pET vector flanking the cloning site. Amplification is carried out using *Taq* DNA polymerase (1 U, Bioline) in *Taq* reaction buffer (as supplied), supplemented with 2 mM MgSO₄ and 0.2 mM dNTPs, using 25 thermal-cycling reactions as detailed above. Successful transformants generated a DNA fragment of approximately 500 bp, ascertained by 1.5 % agarose gel electrophoresis.

### Plasmid DNA preparation

Bacterial transformants that generated the correct size of PCR products are inoculated into 6 ml of sterile LB-kanamycin medium and incubated overnight at 37°C with 200 rpm shaking. pETBMS01 plasmid DNA is recovered from the bacterial cultures using a QIAprep Spin Mini-prep kit according to the manufacturer's instructions (Qiagen). The presence of the β2m fragment in these plasmids is further verified by automated DNA.

A schematic illustration of pETBMS01 is shown in Figure 5(b), specifically illustrating restriction sites.

### 1.2. Cloning the COMP oligomerisation domain into pETBMC01

### Synthesis of the COMP-scFv cassette

The sequence of the oligomerisation domain of COMP is obtained from the Genbank database (accession #1705995) and a region encoding the coiled-coil domain (amino acids 21-85) is selected based on self-association experiments of COMP (Efimov et al., FEBS Letters 341:54-58 (1994)). A linker consisting of 15 amino acids (PQPQPKPQPKPEPET) is incorporated at the C-terminus of the COMP domain to provide spatial separation from the scFv region later on. In addition, the restriction site *Spe* I is created upstream from *Xho* I to allow subsequent insertion of the scFv region.

The whole region is synthesized using the overlapping complementary oligonucleotides (CPL #1, CPL #2, CPL #3, CPL #4, CPL #5 and CPL #6) in a 'PCR assembly' reaction. Oligonucleotide sequences are detailed in Appendix I and are synthesised and purified ('PAGE-pure') by Sigma-Genosys. The primers (4.8 pmoles) are phosphorylated with T4 kinase (10 U, Gibco) for 1 hour at 37°C, in forward reaction buffer (as supplied), supplemented with 1 mM ATP. The kinase reaction is terminated by heating to 80°C for 15 min and then allowed to cool to room temperature, which enables complementary regions to anneal. The annealed oligonucleotides are ligated using T4 DNA ligase as in Section 1.1, and ~10 ng used in a PCR to 'fill in' the ends of the sequences and to amplify the synthetic gene. The PCR reaction is essentially as described in Section 1.1, except CPL #1 (15 µM) and CPL #6 (15 µM) are used as the forward and reverse primers in the following thermal cycling conditions: Step 1: 95 °C, 4 min; Step 2: 95°C, 1 min; Step 3: 70°C, 1 min; Step 4: 72°C, 2 min; Step 5: cycle to Step 2, 15x; Step 6: 72°C, 10 min. A PCR product of the correct size is gel purified as detailed previously (Section 1.1).

### Insertion of COMP-scFv cassette into pETBMS01 to produce pETBMS02

200 ng of purified COMP-scFV and 1 µg pETBMS01 vector are digested for 4 hrs at 37°C using *Hind* III (10 U) and *Xho* I (10 U) restriction enzymes (NEB), as described in Section 1.1. The digestion products are purified, ligated, transformed and PCR screened as in Section 1.1. Plasmids positive from the screen are purified and sequenced as described in Section 1.1.

A schematic diagram of pETBMS02 is shown in Figure 5(c), illustrating restriction sites. The section encoding COMP is out-of-frame in this construct.

### 1.3. Cloning the scFv region into pETBMS02

### Source of anti-CD20 scFv

The sequence of the scFv construct is derived from the anti-CD20 monoclonal antibody clone B9E9, as described in Schultz et al., Cancer Research 60: 6663-6669 (2000). Genbank database accession numbers are #AF277092 for the variable region of the light chain, V_{L}, and #AF277091 for the variable region of the heavy chain, V_{H}. A glycine-serine linker is used to join the two antibody domains, in the order V_{H}-(GGGGS)s-V_{L}. This region is constructed entirely as a synthetic gene in pUC18 vector, using *Spe* I and *Xho* I as flanking restriction sites, as illustrated in Figure 5(f), pUCB9E9 using molecular cloning methods well known in the art. Alternatively, construction of this region can be outsourced to a custom synthetic gene service provider, such as Entelechon GmbH, Regensburg, Germany.

### Insertion of B9E9 scFv synthetic gene into pETBMS02 to produce pETBMS03

1 µg pETBMS02 and 1 µg pUCB9E9 are digested for 4 hrs at 37 ° C using *Spe* I (10U) and *Xho* I (10U) restriction enzymes (NEB), as described in Section 1.1. Digested pUCB9E9 insert and pETBMS02 vector are purified, ligated, transformed and pCR screened as in Section 1.1. Plasmids positive from the screen are purified and sequenced as described in Section 1.1.

A schematic diagram of pETBMS03 is shown in Figure 5(d), illustrating restriction sites. The section encoding COMP-linker-V_{H}-GGGS)₅-V_{L} remains out-of-frame in this construct.

### 1.4. Cloning the poly-glycine linker into pETBMS03

### Synthesis of the poly-glycine linker

The poly-glycine linker connecting the C-terminus of β2m to the N-terminus of COMP is synthesized by annealing overlapping oligonucleotides PGL #1 and PGL #2 (Sigma-Genosys, 'PAGE-pure', Appendix I). The poly-glycine linker comprises the following motif in single letter amino acid code: GS(GGGGS)₄GGK, when encoded in the correct reading frame. The first two residues are encoded by the *BamH* I restriction site, whereas the last residue is encoded by the *Hind* III restriction site. Since the nucleotide sequence of the polyGlycine linker only incorporates the 5' overhang of the cut *BamH* I restriction site, and the 3' overhang of the cut *Hind* III nucleotide recognition motifs, there is no need to digest the annealed product to produce the complementary single-stranded overhangs suitable for subsequent ligation. The oligonucleotides are phosphorylated and annealed as described in Section 1.2.

### Insertion of the poly-glycine linker into pETBMS03 to produce pETBMS04

pETBMS02 is digested with *BamH* I (10U) and *Hind* III (10 U). Ligation of the annealed poly-glycine linker into pETBMS02 was as described previously (Section 1.1), assuming 96 fmoles of annealed oligonucleotide/µl. The transformation and PCR-screening reactions are as described in Section 1.1, but in addition, the presence of an inserted linker is verified by a restriction enzyme digestion of the PCR screen product to ascertain the presence or absence of a *Sal* I restriction site. Successful transformants are not susceptible to *Sal* I digestion, given the removal of the site from the plasmid vector backbone. Purification of pETBMS04 and automated sequencing is as described in Section 1.1.

A schematic diagram of pETBMS04 is shown in Figure 5(e), illustrating restriction sites. The *Bam*H I site encodes a serine residue immediately before the poly-glycine sequence. This serine residue is ultimately removed to give pETBMS05, as illustrated in 5(a).

### 1.5. Deletion of a serine residue immediately prior to the poly-glycine linker

### Site-directed mutagenesis of pETBMS04

Analysis of X-ray crystallography models of MHC class I molecules reveal that the C terminus of β2m closely abuts the α3 domain of the α chain. It is therefore desirable to achieve maximum flexibility at the start of the poly-glycine linker, by removal of the serine residue encoded by the *Bam*H I restriction motif, resulting in a linker comprising G(GGGGS)₄GGK. PCR primers PG_F and PG_R ('PAGE-pure', Appendix I) are obtained, which enabled removal of the TCC triplet that encodes serine within the *Bam* HI recognition motif, using a site-directed mutagenesis kit ('QuickChange', Stratagene) strictly in accordance with the manufacturer's instructions. Corrected versions of this construct are verified by automated sequencing (Section 1.1) and designated pETBMS05. A schematic illustration of pETBMS05 is shown in Figure 5(a). Specifically this figure shows a schematic diagram of the completed β2m-COMP construct in pET24c, illustrating the restriction sites.

### 2. Molecular cloning of the HLA-A*0201 α chain construct

### 2.1. Cloning A*0201 α chain into the pET-11d vector

The extracellular portion of HLA-A*0201 α chain (EMBL M84379) comprises of 276 amino acids (equivalent to Gly1-Pro276 of the mature protein) encoded by bases 73-900 of the messenger RNA sequence. This region of the A*0201 sequence is amplified from a normal human lymphocyte cDNA library by PCR, using the primers A2_F and A2_R which incorporated *Nco* I and *Bam*H I restriction sites respectively. The procedure for cloning the A*0201 insert into *Nco* I/*Bam*H I-digested pET-11d vector (Novagen) is essentially as described for β2m in Section 1.1.

The resulting plasmid is named pETA2sol, and a schematic diagram is shown in Figure 5(g), illustrating restriction sites.

### 3. Expression of β2m-COMP-scFv and HLA-A*0201 α chain proteins

An identical procedure is carried out to produce either β2m-COMP-scFV or A*0201 α chain proteins. Plasmid DNA is transformed into an *E*. *coli* expression host strain in preparation for a large scale bacterial prep. Protein is produced as insoluble inclusion bodies within the bacterial cells, and is recovered by sonication. Purified inclusion bodies are solubilised in denaturing buffer and stored at -80°C until required. Unless specified, chemical reagents are obtained from Sigma.

### 3.1. Large scale production of recombinant proteins

### Transformation of pETBMS05 and pETA2sol into E. coli host strain BL21(DE3)pLysS

Purified plasmid DNA is transformed into the BL21(DE3)pLysS *E*. *coli* strain, which carries a chromosomal copy of the T7 RNA polymerase required to drive protein expression from pET-based constructs. Transformations into BL21(DE3)pLysS competent cells (Stratagene) are carried out with appropriate controls, according to the manufacturer's instructions, using 0.5µg purified plasmid DNA for each reaction. Successful transformants are selected on LB-agar plates containing 34 µg/ml chloramphenicol, in addition to kanamycin 30µg/ml (pETBMC04) or 100µg/ml ampicillin (pETA2sol).

### Growth of E. coli cultures

A single bacterial transformant colony is innoculated into 60ml sterile LB medium, containing appropriate antibiotics for selection, and left to stand overnight in a warm room ( ∼24°C). The resulting overnight culture is added to 6 litres of LB and grown at 37°C with shaking ( ∼240 rpm), up to mid-log phase (OD₆₀₀ = 0.3-0.4). Protein expression is induced at this stage by addition of 1.0 ml of 1M IPTG to each flask. The cultures are left for a further 4 h at 37°C with shaking, after which the cells are harvested by centrifugation and the supernatant discarded.

### Sonication and recovery of proteins

The bacterial cell pellet is resuspended in ice-cold balanced salt solution and sonicated (XL series sonicator; Misonix Inc., USA) in a small glass beaker on ice in order to lyse the cells and release the protein inclusion bodies. Once the cells are completely lysed the inclusion bodies are spun down in 50 ml polycarbonate Oak Ridge centrifuge tubes in a Beckman high-speed centrifuge (J2 series) at 15,000 rpm for 10 min. The inclusion bodies are then washed three times in chilled Triton wash buffer (0. 5 % Triton X-100, 50 mM Tris, pH 8.0, 100 mM sodium chloride, 0.1 % sodium azide and freshly added 2 mM dithiothreitol [DTT]), using a hand-held glass homogeniser to resuspend the pellet after each wash. This is followed by a final wash in detergent-free wash buffer.

The resultant purified protein preparation is solubilised in 20-50 ml of 8 M urea buffer, containing 50 mM MES, pH 6.5, 0.1 mM EDTA and 1 mM DTT, and left on an end-over-end rotator overnight at 4°C. Insoluble particles are removed by centrifugation and the protein yield is determined using Bradford's protein assay reagent (Bio-Rad Laboratories) and by comparison with known standards. The quality of expression and purification is also verified by vertical electrophoresis of samples on a 15 % sodium dodecyl sulphate-polyacrylamide gel (SDS-PAGE). Typical protein yields are 25-75 mg per litre of LB culture. The β2m-COMP and A*0201 α chain proteins are approximately 27 (kD) and 35 kD in size, respectively. Urea-solubilised protein is dispensed in 10mg aliquots and stored at -80°C for future use.

### 4. Formation of pentameric β2m-COMP-scFv complexes

### 4.1. Assembly of β2m-COMP-scFv into pentamers

Assembly of β2m-COMP from the urea-solubilised inclusion bodies is performed by diluting the protein into 20 mM CAPS buffer, pH 11.0, containing 0.2 M sodium chloride and 1 mM EDTA, to give a final protein concentration of 1.5 mg/ml. The protein is oxidised at room temperature by addition of oxidised and reduced glutathione to final concentrations of 20 mM and 2 mM, respectively. Following an overnight incubation, disulphide bond formation is analysed by non-reducing SDS-PAGE on 10 % bis-tricine gels (Invitrogen).

The protein mixture is subsequently buffer exchanged into 20 mM Tris, pH 8.0, 50 mM sodium chloride ('S200 buffer'), using Centricon Plus-20 ultra-centrifugal concentrators (10 kD molecular weight cut-off, Milllipore) according to the manufacturer's instructions, and concentrated to a final volume of 4.5 ml, in preparation for enzymatic biotinylation with BirA (Affinity, Denver, Colorado). 0.5 ml of 10x BirA reaction buffer (as supplied) is added, and recombinant BirA enzyme at 10 µM final concentration, supplemented with 10 mM ATP, pH 7.0. A selection of protease inhibitors is also used to preserve the proteins: 0.2 mM PMSF, 2 µg/ml pepstatin and 2 µg/ml leupeptin. The reaction is left for 4 hours at room temperature.

### 4.2. Purification of β2m-COMP-scFv pentamers

β2m-COMP-scFv pentamers are purified by size exclusion chromatography (SEC) on a Superdex 200 HR 26/60 column (Amersham Biosciences), running S200 buffer as recommended by the manufuacturer. The column is previously calibrated and used according to the manufacturer's instructions and fractions containing pentamers in the region of 135 kD are collected. Protein recovery is determined using the Bradford Assay, and biotinylation efficiency is determined by use of HABA reagent (Pierce), according to manufacturer's instructions.

### 5. Formation of refolded HLA-A*0201/GLCTLVAML complexes

### 5.1. Refolding HLA-A*0201 α chain with biotinylated β2m-COMP and peptide

500 ml of refolding buffer is prepared as follows: 100 mM Tris, pH 8.0, 400 mM L-arginine hydrochloride, 2 mM EDTA, 5 mM reduced glutathione and 0.5 mM oxidised glutathione, dissolved in deionised water and left stirring at 4°C. 15 mg of lyophilised synthetic peptide GLCTLVAML is dissolved in 0.5 ml dimethylsulfoxide and added to the refolding buffer whilst stirring. 50 mg of biotinylated pentameric β2m-COMP and 30 mg of A*0201 α chain is added sequentially, injected through a 23gauge hypodermic needle directly into the vigorously-stirred buffer, to ensure adequate dispersion. The refolding mixture is then left stirring gently for 16 hours at 4°C.

### 5.2. Purification of refolded pentameric HLA-A*0201/GLCTLVAML complexes

The protein refolding mixture is subsequently concentrated from 500 ml to 20 ml using a MiniKros hollow fibre ultrafiltration cartridge (Spectrum Labs, Rancho Dominguez, California) with a 10 kD molecular weight cutoff. Further concentration of the complex from 20 ml to 5 ml is carried out in Centricon Plus-20 centrifugal concentrators (30 kD molecular weight cut-off) according to the manufacturers instructions, followed by buffer exchange into S200 buffer using disposable PD10 desalting columns (Amersham Biosciences), according to the manufacturer's instructions. Final volume is 7.5 ml. The concentrated protein refold mixture is first purified by SEC on a Superdex 200 HR 26/60 gel filtration chromatography column, as in Section 4.2. Fractions containing protein complexes in the region of 310 kD is collected.

Fractions collected from SEC are pooled and subjected to further purification by anion exchange chromatography on a MonoQ HR 5/5 column (Amersham Biosciences), running a salt gradient from 0 - 0.5 M sodium chloride in 20 mM Tris over 15 column volumes. The dominant peak is collected. Protein recovery is determined using the Bradford Assay.

### Appendix I: Table of primers used for synthesis of DNA constructs

| **Primer name** | **Sequence ID No.** | **Direction** | **Sequence (5'-3')** |
|---|---|---|---|
| BM_F | 1 | Forward | GCA TCA CCA TAT GAT CCA GCG TAC TCC AAA GAT TCA GG |
| BM_R | 2 | Reverse | CTA CAA GGA TCC C ATG TCT CGA TCC CAC TTA ACT AT |
| CPL #1 | 3 | Forward | TA AAG CTT CAG GGC CAG AGC CCG TTG GGC TCA GAC CTG GGC CCG CAG ATG CTT CGG GAA CTG CAG GAA ACC AAC GCG GCG |
| CPL #2 | 4 | Reverse | CCT GAC CTG CTG CCG CAG CAG CTC CCG GAC GTC CTG CAG CGC CGC GTT GGT TTC CTG CAG TTC CCG AAG CAT CTG CGG GC |
| CPL #3 | 5 | Forward | CTG CAG GAC GTC CGG GAG CTG CTG CGG CAG CAG GTC AGG GAG ATC ACG TTC CTG AAA AAC ACG GTG ATG GAG TGT GAC GC |
| CPL #4 | 6 | Reverse | CT GGG TAG GCC GGT GCG TAC TGA CTG CTG CAT CCC GCA CGC GTC ACA CTC CAT CAC CGT GTT TTT CAG GAA CGT GAT CTC |
| CPL #5 | 7 | Forward | G TGC GGG ATG CAG CAG TCA GTA CGC ACC GGC CTA CCC AGC GTG CGG CCG CCG CAG CCG CAG CCG AAA CCG CAG CCG AAA C |
| CPL #6 | 8 | Reverse | TA CTC GAG CGC AGA ACC CAG GCT ACT AGT TTC CGG TTC CGG TTT CGG CTG CGG TTT CGG CTG CGG CTG CGG CGG CCG CAC G |
| PGL #1 | 9 | Forward | GAT CCG GTG GTG GTG GTT CTG GTG GTG GTG GTT CTG GTG GTG GTG GTT CTG GTG GTG GTG GTT CTG GTG GTA |
| PGL #2 | 10 | Reverse | AGC TTA CCA CCA GAA CCA CCA CCA CCA GAA CCA CCA CCA CCA GAA CCA CCA CCA CCA GAA CCA CCA CCA CCG |
| PG_F | 11 | Forward | GAG ACA TGG GAG GTG GTG GTG G |
| PG_R | 12 | Reverse | CCA CCA CCA CCT CCC ATG TCT C |
| A2_F | 13 | Forward | GCA TCA CCA TGG GTT CTC ACT CTA TGA GGT ATT TC |
| A2_R | 14 | Reverse | GCA TAC GGA TCC TTA CGG CTC CCA TCT CAG GGT GAG G |
| T7 promoter | 15 | Forward | TAA TAC GAC TCA CTA TAG GG |
| T7 terminator | 16 | Reverse | GCT AGT TAT TGC TCA GCG G |

## Claims

1. Oligomeric MHC complex comprising at least two chimeric proteins, said chimeric proteins comprising a first section comprising an MHC peptide chain and a second section comprising an oligomerising domain from an oligomer-forming coiled-coil protein, wherein the oligomerising domain is from the pentamerisation domain of the cartilage oligomeric matrix protein,
said complex further comprising attaching means for selectively attaching said MHC complex to a target cell,
wherein formation of the oligomeric MHC complex occurs by oligomerisation at the oligomerising domain of the chimeric proteins,
wherein at least two of the first sections are from the same MHC peptide chain,
wherein said attaching means comprises a linking polypeptide with high specific affinity for a target cell specific molecule on the surface of the target cell,
and wherein the first section of the chimeric proteins is from an extra-cellular part of a MHC class I or II α chain or an extra-cellular part of a MHC class I or II β chain.

2. Oligomeric MHC complex of claim 1 wherein the oligomerising domain comprised in the second section in at least one of the chimeric proteins is from the pentamerisation domain of the human cartilage oligomeric matrix protein.

3. Oligomeric MHC complex of one of the preceding claims wherein at least one of the chimeric proteins further comprises a first linker between the MHC peptide chain and the oligomerising domain.

4. Oligomeric MHC complex of one of the preceding claims wherein the attaching means is linked to an oligomerisation domain via a peptide bond or a polypeptide linker.

5. Oligomeric MHC complex of claim 4 wherein said attaching means is comprised in a third section of at least one of the chimeric proteins of the complex.

6. Oligomeric MHC complex of one of the preceding claims, wherein said linking polypeptide comprises a portion of an antibody raised against said target specific molecule.

7. Oligomeric MHC complex of claim 6 wherein said linking polypeptide comprises a variable domain of the binding portion of a monoclonal antibody.

8. Oligomeric MHC complex of claim 6 wherein said linking polypeptide comprises a variable domain and constant domains of the binding portion of a monoclonal antibody.

9. Oligomeric MHC complex of claim 7 or 8 which additionally comprises the complementary domains to the variable domain or constant domains of the binding portion of the monoclonal antibody.

10. Oligomeric MHC complex of claim 6 wherein said linking polypeptide is a single chain variable fragment of a monoclonal antibody.

11. Oligomeric MHC complex of one of the preceding claims wherein the first section is located N-terminal of the second section and the third section, if present, is located C-terminal of said second section.

12. Oligomeric MHC complex of one of the preceding claims wherein at least one of the chimeric proteins further comprises one or more domains selected from the group consisting of a further linker, a tagging domain and a purification domain.

13. Oligomeric MHC complex according to one of the preceding claims further comprising the complementary MHC peptide chains to at least two of the chimeric proteins to form functional MHC binding complexes.

14. Oligomeric MHC complex according to claim 13 further comprising peptide bound to the MHC portions of the complex in the groove formed by the MHC α1 and α2 domains for class I complexes or the MHC α1 and β1 domains for class II complexes.

15. Oligomeric MHC complex according to claim 14 wherein the peptide is substantially homogeneous so that at least 80% of the peptides are identical.

16. Oligomeric MHC complex according to claim 14 or claim 15 wherein the peptide is selected from the group consisting of (a) a peptide against which there is a pre-existing T cell immune response in a patient, (b) a viral peptide, (c) an immunogenic peptide of immunogens used in common vaccination procedures, and (d) a tumour specific peptide, a bacterial peptide, a parasitic peptide or any peptide which is exclusively or characteristically presented on the surface of a diseased, infected or foreign cell.

17. Oligomeric MHC complex according to one of the preceding claims wherein the allotype of the MHC molecules in the complex is different from the allotype of a patient.

18. Oligomeric MHC complex according to one of the preceding claims wherein said target cell is selected from the group of an antigen presenting cell and a CD20 positive cell.

19. Oligomeric MHC complex according to one of the preceding claims comprising a label.

20. Oligomeric MHC complex according to one of the preceding claims for use in amplifying antigen-specific or allospecific T-cells *in vivo* or *in vitro.*

21. Chimeric protein comprising a first section comprising an MHC peptide chain and a second section comprising an oligomerising domain from an oligomer-forming coiled-coil protein which coiled-coil protein oligomerises by alignment of at least two substantially identical versions of the polypeptide chain the oligomerising domain is from and a third section comprising a variable domain from an antibody which antibody is specific for a target molecule present on the surface of a target cell, wherein the oligomerising domain is from the pentamerisation domain of the cartilage oligomeric matrix protein, and wherein the first section of the chimeric proteins is from the extra-cellular part of the MHC class I or II α chain or the extra-cellular part of the MHC class I or II β chain.

22. Chimeric protein according to claim 21 comprising an oligomerising domain from the pentamerisation domain of the human cartilage oligomeric matrix protein.

23. A recombinant expression cassette comprising a promoter sequence operably linked to a nucleotide sequence coding for a chimeric protein as defined in one of claims 21-22.

24. Vector comprising the recombinant expression cassette of claim 23.

25. Pharmaceutical or diagnostic composition, comprising an oligomeric MHC complex according to one of claims 1 to 20.

26. Use of the oligomeric MHC complex of claims 1 to 19 for preparation of a pharmaceutical composition.

## Patentansprüche

1. Oligomerer MHC-Komplex umfassend zumindest zwei chimäre Proteine, wobei diese chimären Proteine einen ersten Abschnitt, der eine MHC-Peptid-Kette umfasst, und einen zweiten Abschnitt umfassen, der eine Oligomerisierungsdomäne von einem Oligomer-bildenden Coiled-coil-Protein umfasst, wobei die Oligomerisierungsdomäne von der Pentamerisierungsdomäne des oligomeren Knorpelmatrixproteins stammt,
wobei dieser Komplex weiter Anheftungsmittel umfasst, um selektiv diesen MHC-Komplex an eine Zielzelle anzuheften,
wobei Bildung des oligomeren MHC-Komplexes durch Oligomerisierung an der Oligomerisierungsdomäne der chimären Proteine erfolgt,
wobei zumindest zwei der ersten Abschnitte von derselben MHC-Peptid-Kette stammen,
wobei dieses Anheftungsmittel ein bindendes Polypeptid mit einer hohen spezifischen Affinität für ein Zielzellen-spezifisches Molekül auf der Oberfläche der Zielzelle umfasst,
und wobei der erste Abschnitt der chimären Proteine von einem extrazellulären Teil einer MHC-Klasse-I- oder -Klasse-II-α-Kette oder einem extrazellulären Teil einer MHC-Klasse-I- oder -Klasse-II-β-Kette stammt.

2. Oligomerer MHC-Komplex nach Anspruch 1, wobei die in dem zweiten Abschnitt in zumindest einem der chimären Proteine umfasste Oligomerisierungsdomäne von der Pentamerisierungsdomäne des menschlichen oligomeren Knorpelmatrixproteins stammt.

3. Oligomerer MHC-Komplex nach einem der vorhergehenden Ansprüche, wobei zumindest eines der chimären Proteine weiter einen ersten Linker zwischen der MHC-Peptid-Kette und der Oligomerisierungsdomäne umfasst.

4. Oligomerer MHC-Komplex nach einem der vorhergehenden Ansprüche, wobei das Anheftungsmittel mittels einer Peptidbindung oder einem Polypeptid-Linker mit einer Oligomerisierungsdomäne verknüpft ist.

5. Oligomerer MHC-Komplex nach Anspruch 4, wobei dieses Anheftungsmittel in einem dritten Abschnitt von zumindest einem der chimären Proteine des Komplexes umfasst ist.

6. Oligomerer MHC-Komplex nach einem der vorhergehenden Ansprüche, wobei dieses bindende Polypeptid einen Teil eines gegen dieses Ziel-spezifische Molekül gerichteten Antikörpers umfasst.

7. Oligomerer MHC-Komplex nach Anspruch 6, wobei dieses bindende Polypeptid eine variable Domäne des bindenden Teils eines monoklonalen Antikörpers umfasst.

8. Oligomerer MHC-Komplex nach Anspruch 6, wobei dieses bindende Polypeptid eine variable Domäne und konstante Domänen des bindenden Teils eines monoklonalen Antikörpers umfasst.

9. Oligomerer MHC-Komplex nach Anspruch 7 oder 8, welcher zusätzlich die komplementären Domänen zu der variablen Domäne oder zu den konstanten Domänen des bindenden Teils des monoklonalen Antikörpers umfasst.

10. Oligomerer MHC-Komplex nach Anspruch 6, wobei dieses bindende Polypeptid ein variables Einzelkettenfragment eines monoklonalen Antikörpers ist.

11. Oligomerer MHC-Komplex nach einem der vorhergehenden Ansprüche, wobei der erste Abschnitt N-terminal von dem zweiten Abschnitt angeordnet ist und der dritte Abschnitt, falls vorhanden, C-terminal von diesem zweiten Abschnitt angeordnet ist.

12. Oligomerer MHC-Komplex nach einem der vorhergehenden Ansprüche, wobei zumindest eines der chimären Proteine weiter eine oder mehrere Domänen, ausgewählt aus der Gruppe, bestehend aus einem weiteren Linker, einer Markierungsdomäne und einer Aufreinigungsdomäne, umfasst.

13. Oligomerer MHC-Komplex nach einem der vorhergehenden Ansprüche, weiter umfassend die zu zumindest zwei der chimären Proteine komplemetären MHC-Peptid-Ketten, um funktionelle MHC-Bindungskomplexe zu bilden.

14. Oligomerer MHC-Komplex nach Anspruch 13, weiter umfassend Peptid, das an die MHC-Teile des Komplexes in der Furche gebunden ist, der durch die MHC-α1- und -α2-Domänen bei Klasse-I-Komplexen oder die MHC-α1- und -β1-Domänen bei Klasse-II-Komplexen gebildet ist.

15. Oligomerer MHC-Komplex nach Anspruch 14, wobei das Peptid im Wesentlichen homogen ist, so dass zumindest 80% der Peptide identisch sind.

16. Oligomerer MHC-Komplex nach Anspruch 14 oder Anspruch 15, wobei das Peptid ausgewählt ist aus der Gruppe, bestehend aus (a) einem Peptid, gegen welches es eine prä-existente T-Zell-Immunantwort in einem Patienten gibt, (b) einem viralen Peptid, (c) einem immunogenen Peptid von in üblichen Vakzinierungsverfahren verwendeten Immunogenen, und (d) einem Tumorspezifischen Peptid, einem bakteriellen Peptid, einem parasitären Peptid oder irgendeinem Peptid, welches ausschließlich oder typischerweise auf der Oberfläche einer kranken, infizierten oder fremden Zelle präsentiert wird.

17. Oligomerer MHC-Komplex nach einem der vorhergehenden Ansprüche, wobei der Allotyp der MHC-Moleküle in dem Komplex sich vom Allotypen eines Patienten unterscheidet.

18. Oligomerer MHC-Komplex nach einem der vorhergehenden Ansprüche, wobei diese Zielzelle aus der Gruppe einer Antigen-präsentierenden Zelle und einer CD20-positiven Zelle ausgewählt ist.

19. Oligomerer MHC-Komplex nach einem der vorhergehenden Ansprüche, umfassend eine Markierung.

20. Oligomerer MHC-Komplex nach einem der vorhergehenden Ansprüche für die Verwendung bei der Amplifizierung von Antigen-spezifischen oder allospezifischen T-Zellen *in vivo* oder *in vitro.*

21. Chimäres Protein, umfassend einen ersten Abschnitt, der eine MHC-Peptid-Kette umfasst, und einen zweiten Abschnitt, der eine Oligomerisierungsdomäne von einem Oligomer-bildenden Coiled-coil-Protein umfasst, welches Coiled-coil-Protein durch Alignment von zumindest zwei im Wesentlichen identischen Versionen der Polypetid-Kette, von welcher die Oligomerisierungsdomäne stammt, oligomerisiert, und umfassend einen dritten Abschnitt, der eine variable Domäne von einem Antikörper umfasst, welcher Antikörper spezifisch für ein auf der Oberfläche einer Zielzelle vorhandenes Zielmolekül ist, wobei die Oligomerisierungsdomäne von der Pentamerisierungsdomäne des oligomeren Knorpelmatrixproteins stammt, und wobei der erste Abschnitt der chimären Proteine von dem extrazellulären Teil der MHC-Klasse-I- oder -Klasse-II-α-Kette oder dem extrazellulären Teil der MHC-Klasse-I- oder -Klasse-II-β-Kette stammt.

22. Chimäres Protein nach Anspruch 21, umfassend eine Oligomersisierungsdomäne von der Pentamerisierungsdomäne des menschlichen oligomeren Knorpelmatrixproteins.

23. Eine rekombinante Expressionskassette, umfassend eine Promotor-Sequenz, die operabel mit einer Nukleotid-Sequenz verknüpft ist, welche für ein chimäres Protein, wie in einem der Ansprüche 21-22 definiert, kodierend ist.

24. Vektor, umfassend die rekombinante Expressionskassette nach Anspruch 23.

25. Pharmazeutische oder diagnostische Zusammensetzung, umfassend einen oligomeren MHC-Komplex nach einem der Ansprüche 1 bis 20.

26. Verwendung des oligomeren MHC-Komplexes der Ansprüche 1 bis 19 für Zubereitung einer pharmazeutischen Zusammensetzung.

## Revendications

1. Complexe oligomère CMH comprenant au moins deux protéines chimères comprenant une première section comprenant une chaîne peptidique CMH et une seconde section comprenant un domaine d'oligomérisation dérivé d'une protéine en forme de superhélice formant un oligomère, le domaine d'oligomérisation étant dérivé du domaine de pentamérisation de la protéine de la matrice oligomère du cartilage,
ledit complexe comprenant de plus des moyens de fixation pour attacher sélective dudit complexe CMH à une cellule cible,
dans lequel formation du complexe oligomère CMH se fait par oligomérisation au niveau du domaine d'oligomérisation des protéines chimères,
dans lequel au moins deux des premières sections sont de la même chaîne peptidique CMH,
dans lequel ledit moyen de fixation comprennent un polypeptide de liaison avec une haute affinité spécifique pour une molécule spécifique de la cellule cible sur la surface de la cellule cible
et dans lequel la première section des protéines chimères est dérivée d'une partie extracellulaire d'une chaîne a du CMH de classe I ou II ou d'une partie extracellulaire d'une chaîne β du CMH de classe I ou II.

2. Complexe oligomère CMH selon la revendication 1 dans lequel le domaine d'oligomérisation compris dans la seconde section dans au moins l'une des protéines chimères est dérivé du domaine de pentamérisation de la protéine de la matrice oligomère du cartilage humain.

3. Complexe oligomère CMH selon l'une des revendications précédentes dans lequel au moins l'une des protéines chimères comprend de plus un premier lieur entre la chaîne peptidique CMH et le domaine d'oligomérisation.

4. Complexe oligomère CMH de l'une des revendications précédentes dans lequel le moyen de fixation est lié à un domaine d'oligomérisation par l'intermédiaire d'une liaison peptidique ou d'un lieur polypeptidique.

5. Complexe oligomère CMH selon la revendication 4 dans lequel le moyen de fixation est compris dans une troisième section d'au moins une des protéines chimères du complexe.

6. Complexe oligomère CMH selon l'une des revendications précédentes dans lequel ledit polypeptide de liaison comprend une partie d'un anticorps dirigé contre ladite molécule cible spécifique.

7. Complexe oligomère CMH selon la revendication 6 dans lequel ledit polypeptide de liaison comprend un domaine variable de la partie de liaison d'un anticorps monoclonal.

8. Complexe oligomère CMH selon la revendication 6 dans lequel ledit polypeptide de liaison comprend un domaine variable et des domaines constants de la partie de liaison d'un anticorps monoclonal.

9. Complexe oligomère CMH selon la revendication 7 ou 8 qui comprend de plus les domaines complémentaires du domaine variable ou des domaines constants de la partie de liaison de l'anticorps monoclonal.

10. Complexe oligomère CMH selon la revendication 6 dans lequel ledit polypeptide de liaison est un fragment variable à chaîne unique d'un anticorps monoclonal.

11. Complexe oligomère CMH selon l'une des revendications précédentes dans lequel la première section est située dans la partie N-terminale de la seconde section et la troisième section, si elle est présente, est située dans la partie C-terminale de ladite seconde section.

12. Complexe oligomère CMH de l'une des revendications précédentes dans lequel au moins une des protéines chimères comprend de plus un ou plusieurs domaines sélectionnés dans le groupe comprenant un autre lieur, un domaine de marquage et un domaine de purification.

13. Complexe oligomère CMH selon l'une des revendications précédentes comprenant de plus les chaînes peptidiques CMH complémentaires à au moins deux des protéines chimères pour former des complexes de liaison CMH fonctionnels.

14. Complexe oligomère CMH selon la revendication 13 comprenant de plus un peptide lié aux portions CMH du complexe dans le sillon formé par les domaines α1 et α2 du CMH pour des complexes de classe I ou des domaines α1 et β1 du CHM pour des complexes de classe II.

15. Complexe oligomère CMH selon la revendication 14 dans lequel le peptide est substantiellement homogène si bien qu'au moins 80% des peptides sont identiques.

16. Complexe oligomère CMH selon la revendication 14 ou la revendication 15 dans lequel le peptide est sélectionné dans le groupe consistant en (a) un peptide contre lequel il y a une réponse immunitaire des cellules T préexistantes dans un patient, (b) un peptide viral, (c) un peptide immunogène d'immunogènes utilisés dans des procédures de vaccination courantes et (d) un peptide de tumeur spécifique, un peptide bactérien, un peptide parasite ou n'importe quel peptide qui est présenté de manière exclusive ou caractéristique sur la surface d'une cellule malade, infectée ou étrangère.

17. Complexe oligomère CMH selon l'une des revendications précédentes dans lequel l'allotype des molécules CMH dans le complexe est différent de l'allotype d'un patient.

18. Complexe oligomère CMH selon l'une des revendications précédentes dans lequel ladite cellule cible est sélectionnée dans le groupe d'une cellule présentant l'antigène et d'une cellule positive CD20.

19. Complexe oligomère CMH selon l'une des revendications précédentes comprenant un marqueur.

20. Complexe oligomère CMH selon l'une des revendications précédentes pour utilisation dans l'amplification de cellules T spécifiques à l'antigène ou allospécifiques *in vivo* ou *in vitro.*

21. Protéine chimère comprenant une première section comprenant une chaîne peptidique CMH et une seconde section comprenant un domaine d'oligomérisation d'une protéine en forme de superhélice qui forme un oligomère, laquelle protéine en forme de superhélice est oligomérisée par alignement d'au moins deux versions substantiellement identiques de la chaîne polypeptidique d'ou vient le domaine d'oligomérisation et une troisième section comprenant un domaine variable à partir d'un anticorps, lequel anticorps est spécifique pour une molécule cible présente sur la surface d'une cellule cible, dans lequel le domaine d'oligomérisation est dérivé du domaine de pentamérisation de la protéine de la matrice oligomère du cartilage et dans lequel la première section des protéines chimères est dérivée de la partie extracellulaire de la chaîne α du CMH de classe I ou II ou de la partie extracellulaire de la chaîne β du CMH de classe I ou II.

22. Protéine chimère selon la revendication 21 comprenant un domaine d'oligomérisation dérivé du domaine de pentamérisation de la protéine de la matrice oligomère du cartilage humain.

23. Cassette d'expression recombinante comprenant une séquence promoteur liée de manière fonctionnelle à une séquence nucléotidique codant pour une protéine chimère comme définie dans l'une des revendications 21-22.

24. Vecteur comprenant la cassette d'expression recombinante selon la revendication 23.

25. Composition pharmaceutique ou de diagnostic comprenant un complexe CMH oligomère selon l'une des revendications 1 à 20.

26. Utilisation du complexe CMH oligomère des revendications 1 à 19 pour la préparation d'une composition pharmaceutique.
